Europäisches Patentamt

European Patent Office

Office européen des brevets

(11) Veröffentlichungsnummer: **0 335 188**

**A1**

# EUROPÄISCHE PATENTANMELDUNG

(21) Anmeldenummer: 89104680.7

(22) Anmeldetag: 16.03.89

(51) Int. Cl.⁴: **B01F 5/02 , C12N 1/06**

(30) Priorität: 26.03.88 DE 3810462

(43) Veröffentlichungstag der Anmeldung:
04.10.89 Patentblatt 89/40

(84) Benannte Vertragsstaaten:
AT BE CH DE ES FR GB GR IT LI LU NL SE

(71) Anmelder: DECHEMA Deutsche Gesellschaft
für Chemisches Apparatewesen, Chemische
Technik und Biotechnologie e.V.
Theodor-Heuss-Allee 25
D-6000 Frankfurt am Main 97(DE)

(72) Erfinder: Krämer, Peter, Dr.-Ing.
Ahornweg 12
D-6236 Eschborn 2(DE)
Erfinder: Bomberg, Andreas Rolf, Dipl. Biol.
Marktplatz 5
D-6453 Seligenstadt(DE)
Erfinder: Gödelmann, Bernhard
Königsteiner Strasse 83
D-6230 Frankfurt am Main 80(DE)

(74) Vertreter: Beil, Hans Chr., Dr. et al
Beil, Wolff und Beil, Rechtsanwälte
Adelonstrasse 58 Postfach 80 01 40
D-6230 Frankfurt am Main 80(DE)

(54) Verfahren zum Aufschluss von Zellen in einer Zellsuspension und Vorrichtung zur Durchführung des Verfahrens.

(57) Verfahren zum Aufschluss von Zellsuspensionen durch Eindüsen von zwei Teilströmen der Suspension in eine Zellkammer mit einer Düsenaustrittsgeschwindigkeit von je mindestens 75 m/s, wobei die Düsen der Aufschlusskammer genau gegeneinander gerichtet, miteinander fluchtend und in geringem Abstand voneinander angeordnet sind, sowie Vorrichtung zur Durchführung dieses Verfahrens.

EP 0 335 188 A1

## Verfahren zum Aufschluss von Zellen in einer Zellsuspension und Vorrichtung zur Durchführung des Verfahrens

Die vorliegende Erfindung betrifft ein Verfahren zum Aufschluss von Zellen in einer Suspension sowie die dafür geeignete Vorrichtung.

Die Zerstörung der Zellwand, insbesondere von Mikroorganismen, der sogenannte Zellaufschluss, ist in Verbindung mit der Zellernte der erste Verfahrensschritt zur Isolierung intrazellulärer Produkte. Dieser Zellaufschluss gewinnt immer mehr an Bedeutung, seit es durch die Fortschritte der Gentechnologie zunehmend möglich geworden ist, artfremde Proteine in Bakterien und/oder Hefen zu produzieren. Zu diesen intrazellulär erzeugten Produkten gehören immer mehr auch höhermolekulare Substanzen, wie Enzyme oder Proteine humanen Ursprungs. Die Entwicklung von Enzymreaktoren und von Methoden der kontinuierlichen Coenzymregenerierung trägt ebenfalls dazu bei, dass der Gewinnung intrazellulärer, biologisch aktiver Stoffe eine grosse Bedeutung zukommt.

Für solche Aufschlussverfahren kommen chemische, physikalische, biologische und mechanische Methoden in Betracht. Erwünscht ist im Hinblick darauf, dass intrazelluläre Produkte einen teuren Rohstoff darstellen, ein möglichst vollständiger Aufschluss der Biomasse unter weitestgehender Schonung der oftmals gegen Temperaturen, Scherkräfte und pH-Wert-Schwankungen empfindlichen intrazellulären Produkte.

Chemische Aufschlussverfahren haben den Nachteil, dass die angewandten Aufschlussmittel, wie Lösungsmittel, Säuren oder Laugen, nach dem Aufschluß schritt wieder vollständig abgetrennt werden müssen, was oftmals grossen Schwierigkeiten begegnet. Biologische Aufschlüsse mit Enzymen oder Phagen verbieten sich häufig deshalb, weil einerseits die eingesetzten Aufschlussmittel ihrerseits sehr teuer und andererseits die intrazellulären Produkte gegen solche Aufschlussmittel nicht resistent sind. Physikalische Methoden, wie etwa Einfrieren und Wiederauftauen oder die Einwirkung von osmotischem Druck, sind oftmals kontinuierlich nur schwer durchführbar.

Als mechanische Aufschlussverfahren sind insbesondere die Behandlung mit Ultraschall, das Nassvermahlen in Rührwerkkugelmühlen und die Hochdruckhomogenisation bekannt.

Die Behandlung mit Ultraschall ist zwar im Labor eine geeignete Massnahme, versagt jedoch im technischen Maßstab, insbesondere auch wegen der dabei auftretenden Erwärmung der Suspension, weitgehend. Bei einer Nassvermahlung in schnellaufenden Rührwerkkugelmühlen im technischen Maßstab ergeben sich erhebliche technische Schwierigkeiten. Mit zunehmendem Apparatevolumen müssen grössere Fertigungs toleranzen in Kauf genommen werden. Dies führt beispielsweise bei Wellendurchführungen in Rührwerkkugelmühlen zu Problemen, insbesondere im Hinblick auf eine sterile Betriebsführung und den verstärkten Dichtungsverschleiss, bedingt durch die in sehr kleinen Abmessungen eingesetzten Mahlkörper mit Dimensionen zwischen 0,25 und 1,0 mm, die nach dem Aufschluss auch schwierig abzutrennen sind. Ferner gestaltet sich die Wärmeabfuhr bei mit hohen Energiedissipationsdichten operierenden Methoden zunehmend problematischer, da die auf das Apparatevolumen bezogene Mantelfläche immer kleiner wird, die Apparate aber meistens mit Mantelkühlung betrieben werden müssen.

Eine weitere Aufschlussmethode bedient sich der Hochdruckhomogenisation, die ursprünglich aus der Milchverarbeitung stammt. Dabei wird die Zellsuspension mit einer Hochdruckkolbenpumpe in eine Ventileinheit gepumpt. Das Ventil öffnet erst nach Überschreiten eines über ein Hydrauliksystem je nach Anforderung einstellbaren Gegendrucks von etwa 500 - 1000 bar. Die Suspension wird gegen einen Prallring geschleudert und nach Umlenkung um 90° abgeleitet.

Ebenfalls in Druckbereichen wie bei der Hochdruckhomogenisation arbeiten Vorrichtungen, wie sie aus US-PS 4 533 254 bekannt und im Handel sind. Die Spaltbreiten der rechteckigen Strömungskanäle liegen dort um 10μm x 1000μm und der Abstand der Austrittsöffnungen beträgt 0,2 mm. Ihr Nachteil liegt insbesondere darin, dass einerseits sehr hohe Betriebsdrucke (bis zu 1200 bar) erforderlich sind, die Durchflussgeschwindigkeit nur über apparativen Umbau variiert und somit zur Aufschlussoptimierung nicht herangezogen werden kann und die Düsen leicht verstopfen, was einerseits eine Fein-Vorfiltration der Suspension erforderlich macht und andererseits oftmalige Stillstandzeiten für die schwierige Düsenreinigung erfordert. Ausserdem tritt eine Erwärmung der Suspension ein, die bei einem handels üblichen Gerät nach Angaben des Herstellers im Bereich von 2,0 -2,4° C/100 bar liegt und in Einzelfällen bei hohen Betriebsdrücken zu einer Schädigung der intrazellulären Produkte führen kann.

Aufgabe der vorliegenden Erfindung ist es, ein Aufschlussverfahren bereitzustellen, das die Nachteile des Standes der Technik vermeidet, einfach zu handhaben ist und weder auf später abzutrennende Hilfsmittel noch auf extreme Verfahrensbedingungen angewiesen ist. Erfindungsgemäss wird diese Aufgabe dadurch gelöst, dass man die Suspension der aufzuschliessenden Zellen in zwei Teilströmen aus zwei gegeneinander gerichteten, miteinander genau fluchtenden und im geringen Abstand voneinander angeordneten Düsen in eine Aufschlusskammer eindüst, wobei die Düsenaustrittsgeschwindigkeit jedes Teilstroms mindestens 75 m/s beträgt.

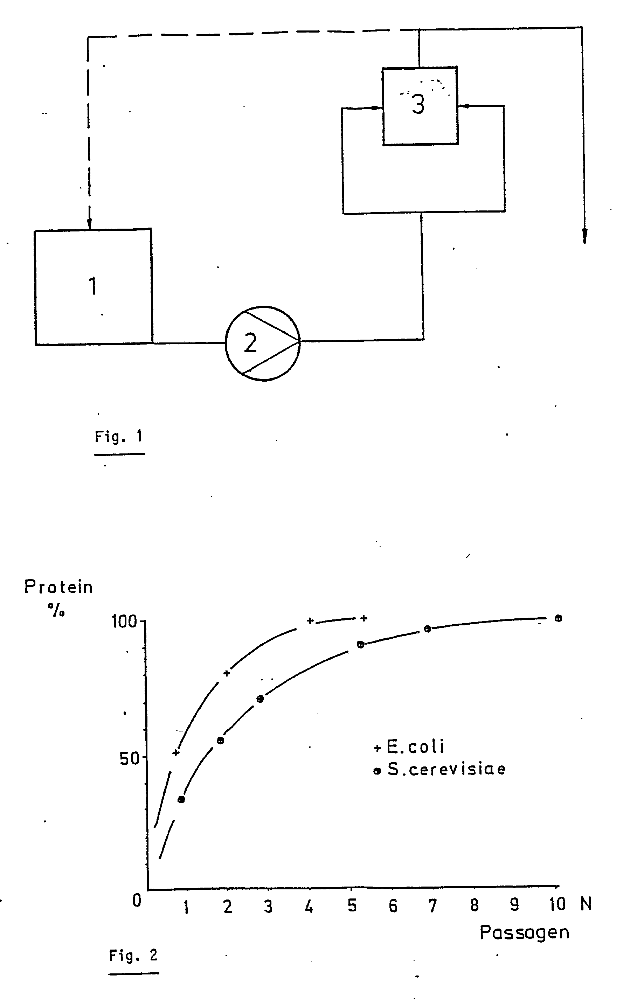

Praktisch wird das Aufschlussverfahren, wie anhand des Fließschemas der Figur 1 erläutert wird, in der Weise durchgeführt, dass man die aufzuschliessenden Zellen in einem geeigneten Medium, wie beispielsweise einem Phosphat-Puffer (Zusammensetzung: 50 mM $KH_2PO_4$, 50 mM $K_2HPO_4$ und 0,2 mM Dithioerythritol, pH = 7,0) suspendiert, die Suspension aus einem Vorratsbehälter (1) durch eine geeignete Pumpe (2) in zwei sich hinter der Pumpe teilenden, gleichgrossen Teilströmen zu der Aufschlusskammer (3) pumpt und durch die in dieser Aufschlusskammer angebrachten Düsen mit den Austrittsstrahlen der Teilströme aufeinandertreffen lässt. Dabei ist es nicht erforderlich, dass die Aufschlusskammer stets völlig leer ist, vielmehr können die Teilströme auch in eine mit schon eingedüster Zellsuspension gefüllte Kammer eingedüst werden. Falls erwünscht, kann die Suspension ganz oder teilweise für eine erneute Passage dem Vorratsbehälter (1) oder einem gesonderten Behälter zugeführt werden.

Die für einen Aufschluss notwendige Düsenaustrittsgeschwindigkeit der Teilströme liegt im Bereich von oberhalb 70 m/s. Die zur Erzielung dieser Geschwindigkeiten erforderlichen Drücke liegen in Abhängigkeit von der Düsengeometrie oberhalb von etwa 130 bar. Der Abstand der Düsenöffnungen hängt von der Grösse der Düsenöffnungen ab und kann durch einfache Versuche leicht optimiert werden. Ist der Abstand der Düsenöffnungen zu klein, so tritt ein Rückstau ein, weil die eingedüste Suspension nicht schnell genug seitlich ausweichen kann. Dies muss auf jeden Fall vermieden werden. Bei einer Düsenöffnung von 0,2 mm muss der Düsenabstand mindestens 2 mm betragen. Beträgt der Durchmesser der Düsenöffnung 1 mm, so ist ein Düsenabstand in der Grössenordnung von etwa 60 mm notwendig.

Der Grad des Aufschlusses der Zellen hängt entscheidend von der Art der Zellen und von der Düsenaustrittsgeschwindigkeit ab, wie sich aus der nachfolgenden Tabelle 1 ergibt.

## TABELLE I

Aufschlussgrad in Abhängigkeit von der Düsenaustrittsgeschwindigkeit der Zellsuspension bei einer Passage für die Mikroorganismen:

a) E.coli K 12 (DSM 498)

| Geschwindigkeit [m/s] | 75 | 84 | 90 | 95 | | |
|---|---|---|---|---|---|---|
| Aufschlussgrad % | 11 | 27 | 52 | 64 | | |

b) S.cerevisiae (Bäckerhefe Fa.Pleser, Darmstadt)

| Geschwindigkeit [m/s] | 71 | 75 | 84 | 95 | | |
|---|---|---|---|---|---|---|
| Aufschlussgrad % | 4 | 7 | 17 | 34 | | |

c) S.coli (rDNA-Stamm)

| Geschwindigkeit [m/s] | 110,5 | 123,5 | 128,5 | 140 | 157 | 162,5 |
|---|---|---|---|---|---|---|
| Aufschlussgrad % | 50 | 60 | 70 | 90 | 95 | 99,9 |

Wenn im Einzelfall der Druck bzw. die Austrittsgeschwindigkeit der Teilströme für einen ausreichenden Aufschluss bei einmaligem Durchgang nicht ausreicht, ermöglichen Mehrfachpassagen eine deutliche Verbesserung des Aufschlussergebnisses, wie aus Figur 2 ersichtlich ist, worin N die Zahl der Passagen der untersuchten beiden Zellsuspensionen bezeichnet.

Dauerversuche mit Fumarase und saurer Phosphatase in einer Pufferlösung, welche ausserdem noch Zellfragmente enthielt, führten darüberhinaus zu der überraschenden Erkenntnis, dass selbst bei einer Vielzahl von Passagen kein messbarer (Fumarase) bzw. nur sehr geringer (s.Phosphatase) Enzymaktivitätsverlust eintrat, was den Schluss zulässt, dass eine Enzymschädigung ausbleibt (fumarase) bzw. nur sehr gering ist (s.Phosphatase) und das Aufschlussverfahren daher äusserst schonend ist.

Die Verfahrensoptimierung für den Aufschluss bestimmter Zellen oder Mikroorganismen im Rahmen der erfindungsgemässen Parameter des Verfahrens und Vorrichtungsmerkmale liegt im Bereich des Fachwissens und erfordert nur einfache Versuche.

Das erfindungsgemässe Verfahren eignet sich auch zum Aufschluss von Mischkulturen, wie beispielsweise den Aufschluss von Klärschlamm mit dem Ziel der Freisetzung von intrazellulär gebundenem Wasser.

## Ansprüche

1. Verfahren zum Aufschluss von Zellen in Zellsuspensionen dadurch gekennzeichnet, dass man die Zellsuspension in zwei gleichgrossen Teilströmen durch zwei gegeneinander gerichtete, miteinander fluchtende und in geringem Abstand voneinander angeordnete Düsen mit einer Düsenaustrittsgeschwindigkeit von je mindestens 75 m/s in eine Aufschlusskammer eindüst.

2. Verfahren nach Anspruch 1, dadurch gekennzeichnet, dass die Durchflussgeschwindigkeit der Teilströme 80- 300, vorzugsweise 100 - 200 m/s beträgt.

3. Verfahren nach Anspruch 1 oder 2, dadurch gekennzeichnet, dass die Zellsuspension mehrfach durch die Düsen geleitet wird.

4. Vorrichtung zur Durchführung des Verfahrens nach Anspruch 1 - 3, bestehend aus

(a) einem Vorratsbehälter für die Zellsuspension,

(b) einer Förderpumpe zum Transport der Zellsuspension,

(c) einer Aufschlusskammer mit zwei gegeneinander gerichteten, genau miteinander fluchtenden und in geringem Abstand voneinander im Inneren der Kammer angeordneten Düsen,

(d) einer Verbindungsleitung von dem Vorratsbehälter zu der Förderpumpe und von der Förderpumpe zu den beiden Düsen,

(e) einer Entnahmestelle für die aufgeschlossene Zellsuspension und

(f) gegebenenfalls einer Rückflussleitung von der Aufschlusskammer zum Vorratsbehälter.

5. Vorrichtung nach Anspruch 4, dadurch gekennzeichnet, dass die Düsen eine Öffnung mit einem Durchmesser von 0,2 bis 1,0 mm aufweisen und in Abhängigkeit von dem Durchmesser der Düsenöffnung in einem Abstand von 2 bis 60 mm angeordnet sind.

Fig. 1

+E.coli
⊕ S.cerevisiae

Fig. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | FR-A- 346 862 (EMPIS) <br> * Seite 1, Zeilen 39-50; Seite 2, Zusammenfassung * <br> --- | 1,2 | B 01 F 5/02 <br> C 12 N 1/06 |
| Y | US-A-2 976 024 (MARTINEK) <br> * Spalte 4, Zeilen 26-30; Figuren * <br> --- | 1,2 | |
| A,D | US-A-4 533 254 (COOK) <br> * Spalte 5, Zeile 49 - Spalte 6, Zeile 54; Spalte 8, Zeilen 13-23; Figuren * <br> --- | 1-5 | |
| A | US-A-2 751 425 (RUPP) <br> * Spalte 9, Zeilen 13-23; Figuren * <br> --- | 5 | |
| A | GB-A-2 063 695 (KONISHIROKU) <br> * Seite 1, Zeilen 100-104; Figuren * <br> --- | | |
| A | US-A-3 391 908 (MacDONALD) <br> --- | | |
| A | US-A-2 751 335 (CARVER) <br> --- | | |
| A | CHEMICAL ABSTRACTS, Band 79, Nr. 21, 26. November 1973, Seite 272, Zusammenfassung Nr. 124689g, Columbus, Ohio, US; & JP-A-72 43 834 (KANEGAFUCHI CHEMICAL INDUSTRY CO., LTD) 06-11-1972 <br> ----- | | |

| | |
|---|---|
| | RECHERCHIERTE SACHGEBIETE (Int. Cl.4) |
| | B 01 F |

Der vorliegende Recherchenbericht wurde für alle Patentansprüche erstellt

| Recherchenort | Abschlußdatum der Recherche | Prüfer |
|---|---|---|
| DEN HAAG | 23-06-1989 | PEETERS S. |